# EUROPEAN PATENT SPECIFICATION

(11) **EP 2 635 334 B1**
(45) Date of publication and mention of the grant of the patent: **13.08.2014**
(21) Application number: 11779380.2
(22) Date of filing: 31.10.2011
(51) Int. Cl.: A61M 5/32, A61M 5/20, A61M 5/50, A61M 5/31

(54) **SYSTEM FOR PREVENTING LEAKAGE OF A MEDICAMENT**
SYSTEM ZUR VERHINDERUNG DES AUSLAUFENS EINES MEDIKAMENTS
SYSTÈME PERMETTANT D'EMPÊCHER LA FUITE D'UN MÉDICAMENT

(30) Priority: 03.11.2010 EP 10189773
(43) Date of publication of application: 11.09.2013
(73) Proprietor: Sanofi-Aventis Deutschland GmbH, 65929 Frankfurt am Main (DE)
(72) Inventor: DAVIES, James Alexander, Leamington Spa Warwickshire CV32 7XB (GB); WIMPENNY, Steven, Leamington Spa Warwickshire CV32 6ES (GB)
(74) Representative: Keil & Schaafhausen Patent- und Rechtsanwälte
(86) International application number: PCT/EP2011/069092
(87) International publication number: WO 2012/059449

(56) References cited:
- WO-A1-95/32015
- WO-A1-2010/072644
- US-A- 3 946 732
- US-B1- 6 562 002

## Description

### Field of the Present Patent Application

This present patent application relates to medical devices and methods of delivering at least two drug agents from separate reservoirs using devices having only a single dose setting mechanism and a single dispense interface. A single delivery procedure initiated by the user causes a non-user settable dose of a second drug agent and a variable set dose of a first drug agent to be delivered to the patient. The drug agents may be available in two or more reservoirs, containers or packages, each containing independent (single drug compound) or pre-mixed (co-formulated multiple drug compounds) drug agents. Specifically, this application concerns a method and system for preventing leakage of a medicament in a medicated module that is used to deliver the non-user settable dose of the second drug agent.

### Background

Certain disease states require treatment using one or more different medicaments. Some drug compounds need to be delivered in a specific relationship with each other in order to deliver the optimum therapeutic dose. The presently proposed devices and methods are of particular benefit where combination therapy is desirable, but not possible in a single formulation for reasons such as, but not limited to, stability, compromised therapeutic performance and toxicology.

For example, in some cases it might be beneficial to treat a diabetic with a long acting insulin and with a glucagon-like peptide-1 (GLP-1), which is derived from the transcription product of the proglucagon gene. GLP-1 is found in the body and is secreted by the intestinal L cell as a gut hormone. GLP-1 possesses several physiological properties that make it (and its analogs) a subject of intensive investigation as a potential treatment of diabetes mellitus.

US 6,562,002 B1 discloses a single dose delivery device that combines a micro-injection device comprising a housing, which is shaped and sized to house a reagent bed and a porous compression component and which has a distal injection needle, with a diluent delivery device. The diluent delivery device comprises a connecting needle extending from a proximal end to a distal end. When the micro-injection device is attached to the diluent delivery device, the proximal needle end opens fluid communication to a diluent chamber and the distal needle end opens fluid communication to the reagent chamber in the micro-injection device. During delivery, diluent flows from the diluent delivery device through the needle into the reagent chamber of the micro-injection device. Passage of the diluent through the reagent bed results in dissolution of the reagent. The reagent solution is provided by the injection needle.

There are a number of potential problems that can arise when delivering two active medicaments or "agents" simultaneously. The two active agents may interact with each other during the long-term, shelf life storage of the formulation. Therefore, it is advantageous to store the active components separately and combine them at the point of delivery, e.g. injection, needle-less injection, pumps, or inhalation. However, the process for combining the two agents needs to be simple and convenient for the user to perform reliably, repeatedly and safely.

A further problem is that the quantities and/or proportions of each active agent making up the combination therapy may need to be varied for each user or at different stages of their therapy. For example one or more active agents may require a titration period to gradually introduce a patient up to a "maintenance" dose. A further example would be if one active agent requires a non-adjustable fixed dose while the other is varied in response to a patient's symptoms or physical condition. This potential problem means that pre-mixed formulations of multiple active agents may not be suitable as these pre-mixed formulations would have a fixed ratio of the active components, which could not be varied by the healthcare professional or user.

Additional problems arise where a multi-drug compound therapy is required, because many users cannot cope with having to use more that one drug delivery system or make the necessary accurate calculation of the required dose combination. This is especially true for users with dexterity or computational difficulties. In some circumstances it is also necessary to perform a priming procedure of the device and/or needle cannulae before dispensing the medicaments. Likewise, in some situations, it may be necessary to bypass one drug compound and to dispense only a single medicament from a separate reservoir.

Accordingly, there exists a need to provide devices and methods for the delivery of two or more medicaments in a single injection or delivery step that is simple for the user to perform. The presently proposed devices and methods overcome the above-mentioned problems by providing separate storage containers for two or more active drug agents that are then only combined and/or delivered to the patient during a single delivery procedure. Setting a dose of one medicament automatically fixes or determines the dose of the second medicament (i.e. non-user settable). The proposed devices and methods also give the opportunity for varying the quantity of one or both medicaments. For example, one fluid quantity can be varied by changing the properties of the injection device (e.g. dialing a user variable dose or changing the device's "fixed" dose). The second fluid quantity can be changed by manufacturing a variety of secondary drug containing packages with each variant containing a different volume and/or concentration of the second active agent. The user or healthcare professional would then select the most appropriate secondary package or series or combination of series of different packages for a particular treatment regime. The proposed medicated module forms a self-contained reservoir in which non-user-settable dose of a medicament may be stored. The proposed medicated module also is designed so as to prevent leakage of the medicament from the medicated module.

These and other advantages will become evident from the following more detailed description. A medicated module according to the invention is defined in claims 1 and 9.

### SUMMARY

The presently proposed devices and methods allow for complex combinations of multiple drug compounds within a single drug delivery system. The presently proposed devices and methods allow the user to set and dispense a multi-drug compound device through one single dose setting mechanism and a single dispense interface. This single dose setter controls the mechanism of the device such that a predefined combination of the individual drug compounds is delivered when a single dose of one of the medicaments is set and dispensed through the single dispense interface.

By defining the therapeutic relationship between the individual drug compounds, the proposed delivery device and delivery methods help ensure that a patient/user receives the optimum therapeutic combination dose from a multi-drug compound device without the inherent risks associated with multiple inputs where the user has to calculate and set the correct dose combination every time they use the device. The medicaments can be fluids, defined herein as liquids or gases or powders that are capable of flowing and that change shape at a steady rate when acted upon by a force tending to change its shape. Alternatively, one or both of the medicaments may be a solid that is carried, solubilized or otherwise dispensed with another fluid medicament.

Applicants' proposed concept is of particular benefit to users with dexterity or computational difficulties as the single input and associated predefined therapeutic profile removes the need for them to calculate their prescribed dose every time they use the device and the single input allows considerably easier setting and dispensing of the combined compounds.

In a preferred embodiment a master drug compound, such as insulin, contained within a multiple dose, user selectable device could be used with a single use, user replaceable, module that contains a single dose of a secondary medicament and the single dispense interface. When connected to the primary device, the secondary compound is activated/delivered on dispense of the primary compound.

Although the present application specifically mentions insulin, insulin analogs or insulin derivatives, and GLP-1 or GLP-1 analogs as two possible drug combinations, other drugs or drug combinations, such as an analgesics, hormones, beta agonists or corticosteroids, or a combination of any of the above-mentioned drugs could be used with our invention.

For the purposes of our invention the term "insulin" shall mean Insulin, insulin analogs, insulin derivatives or mixtures thereof, including human insulin or a human insulin analogs or derivatives. Examples of insulin analogs are, without limitation, Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin or Des(B30) human insulin. Examples of insulin derivatives are, without limitation, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta-decanoyl) human insulin.

As used herein the term "GLP-1" shall mean GLP-1, GLP-1 analogs, or mixtures thereof, including without limitation, exenatide (Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2), Exendin-3, Liraglutide, or AVE0010 (H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-Lys-Lys-Lys-Lys-Lys-Lys-NH2).

Examples of beta agonists are, without limitation, salbutamol, levosalbutamol, terbutaline, pirbuterol, procaterol, metaproterenol, fenoterol, bitolterol mesylate, salmeterol, formoterol, bambuterol, clenbuterol, indacaterol.

Hormones are for example hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

According to an embodiment, according to claim 1, a medicated module is attachable to a drug delivery device. The medicated module includes a needle, and this needle holds a medicament. The needle includes an engagement needle portion and an output needle portion in fluid communication with each other. The medicated module further includes a first seal and a second seal. A proximal end of the engagement needle portion partially pierces the first seal, and a distal end of the output needle portion partially pierces the second seal.

According to another embodiment, according to claim 9, a medicated module includes an engagement needle and an output needle, wherein the engagement needle and the output needle are in fluid communication. Further, the engagement needle and the output needle hold a medicament. The medicated module also includes a first seal and a second seal. A proximal end of the engagement needle partially pierces the first seal, and a distal end of the output needle partially pierces the second seal. Other embodiments include a drug delivery device including such medicated modules.

The medicated module can be designed for use with any drug delivery device with an appropriate compatible interface. However, it may be preferable to design the module in such a way as to limit its use to one exclusive primary drug delivery device (or family of devices) through employment of dedicated or coded features to prevent attachment of a non-appropriate medicated module to a non-matching device. In some situations it may be beneficial to ensure that the medicated module is exclusive to one drug delivery device while also permitting the attachment of a standard drug dispense interface to the device. This would allow the user to deliver a combined therapy when the module is attached, but would also allow delivery of the primary compound independently through a standard drug dispense interface in situations, such as, but not limited to, dose splitting or top-up of the primary compound.

A particular benefit of Applicants' method and system is that the method and system provides a fluid seal to the ends of needles in a medicated module in order to prevent leakage of a medicament in the medicated module. The fluid seals remain sealed until either attachment to a drug delivery device or just prior to injection of the medicament. Beneficially, the fluid seal may also reduce or prevent contamination of the medicament.

In a preferred embodiment, the primary drug delivery device is used more than once and therefore is a multi-use device; however, the drug delivery device may also be a single use disposable device. Such a device may or may not have a replaceable reservoir of the primary drug compound, but our proposed concept is equally applicable to both scenarios. It is also possible to have a suite of different medicated modules for various conditions that could be prescribed as one-off extra medication to patients already using a standard drug delivery device. Should the patient attempt to reuse a previously used medicated module, this module may include a locking needle guard that is activated after a user delivers a dose from the medicated module. Other means of alerting the user may include some (or all) of the following:
Physical prevention of medicated module re-attachment to the primary drug deliver device once the module has been used and removed.
Physical / hydraulic prevention of subsequent liquid flow through the drug dispense interface once it has been used.
Physical locking of the dose setter and/or dose button of the primary drug delivery device.
Visual warnings (e.g. change in color and/or warning text/indicia within an indication window on the module once insertion and/or fluid flow has occurred).
Tactile feedback (presence or absence of tactile features on the outer surface of the module hub following use).

A further proposed feature is that both medicaments are delivered via one injection needle and in one injection step. This offers a convenient benefit to the user in terms of reduced user steps compared to administering two separate injections. This convenience benefit may also result in improved compliance with the prescribed therapy, particularly for users who find injections unpleasant or who have computational or dexterity difficulties.

These as well as other advantages of various aspects of the present invention will become apparent to those of ordinary skill in the art by reading the following detailed description, with appropriate reference to the accompanying drawings.

### BRIEF DESCRIPTION OF THE DRAWINGS

Exemplary embodiments are described herein with reference to the drawings, in which:
Figure 1 illustrates a perspective view of one possible drug delivery device that can be used with Applicants' medicated module;
Figure 2 illustrates a cross-sectional view of an exemplary medicated module;
Figure 3 illustrates a cross-sectional view of the exemplary medicated module of Figure 2 attached to an exemplary drug delivery device; and
Figure 4 illustrates a cross-sectional view of an exemplary medicated module attached to an exemplary drug delivery device.

### DETAILED DESCRIPTION

Applicants' proposed concept is a system and method for preventing leakage of a medicament in a medicated module. The proposed concept specifically relates to sealing features of a medicated module, where the medicated module is intended to be used in conjunction with a primary injection device to deliver a fixed dose of a secondary medicament when a user injects a selected dose of a primary medicament.

A medicated module in accordance with embodiments of Applicants' proposed concepts may be attached to a primary drug delivery device, such as drug delivery device 100. Generally, Applicants' proposed arrangement includes a series of movable seals that are used to seal the medicament stored in the medicated module. The medicament is stored in a needle (or in a first and second needle). Ends of the needle (or an end of the first needle and an end of the second needle) partially pierce the seals prior to attachment to a drug delivery device and/or insertion. In this condition of partially pierced seals, fluid-tight seals are provided and maintained in the medicated module. However, movement of the seals eventually removes the fluid-tight seals. For example, a top seal may be moved due to attachment to the primary drug delivery device. This movement causes a needle tip to fully pierce the top seal, thereby allowing fluid communication between the first and second medicament to occur. Further, a bottom seal may be moved prior to or just prior to insertion. This movement removes the bottom seal from the needle tip of the output needle, and the output needle may then be used to subcutaneously inject both the first and second medicament.

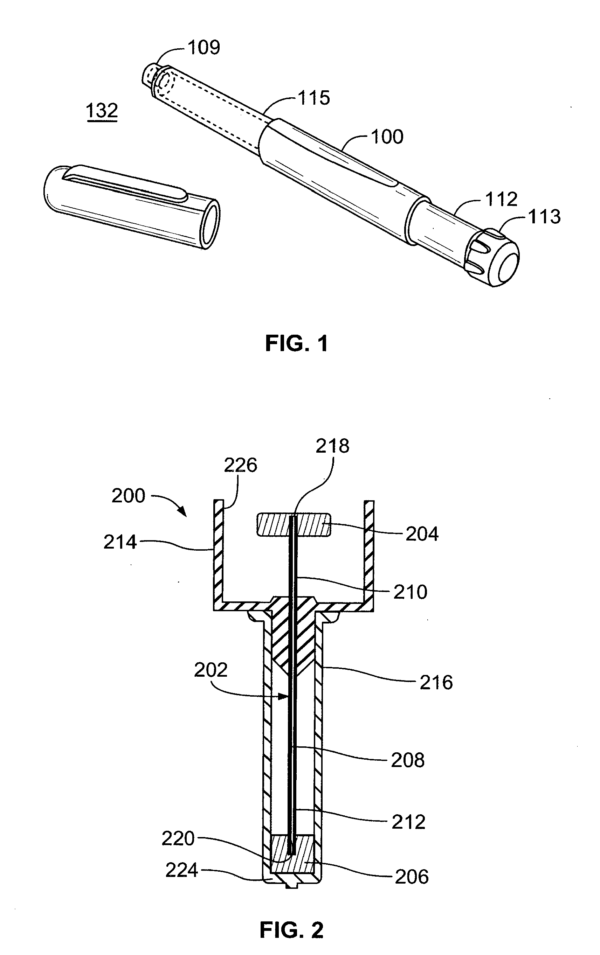

Figure 1 illustrates one example of a drug delivery device 100 that a medicated module, such as the medicated modules depicted in Figures 2-4, can be attached to the connection means 109 of distal end 132. Each medicated module is preferably self-contained and provided as a sealed and sterile disposable module that has an attachment means compatible to the attachment means 109 at the distal end 132 of device 100. Although not shown, the medicated module could be supplied by a manufacturer contained in a protective and sterile container, where the user would peel or rip open a seal or the container itself to gain access to the sterile medicated module. Further, the drug delivery device 100 includes a housing including a single dose setter 112. The dose setter 112 may be operably connected to a primary reservoir of medicament that may be stored in the drug delivery device, such as in cartridge holder 115. The user may use a dose dial button 113 in order to dial a user selectable dose of the primary medicament.

Applicants' proposed concept is a sealing means (e.g., sealing features) for a medicated module that can attach to a drug delivery device. In accordance with an embodiment of Applicants' proposed concept, a medicated module includes a needle that holds medicament. The needle includes an engagement needle portion and an output needle portion, and the engagement needle portion and output needle portion are in fluid communication with each other. The medicated module also includes a first seal and a second seal. Prior to attaching the medicated module to a drug delivery device, a proximal end of the engagement needle portion partially pierces the first seal, and a distal end of the output needle partially pierces the second seal. The seals provide fluid-tight seals that seal the medicament in the needle. In another embodiment, rather than having a single needle that holds medicament, a medicated module may include two needles (i.e., an engagement needle and an output needle) that are in fluid communication with one another and that hold the medicament.

Figure 2 illustrates a medicated module 200 that includes sealing features in accordance with Applicants' proposed concept. The medicated module 200 includes a needle 202, a first seal 204, and a second seal 206. The needle 202 holds a medicament 208. Further, the needle has an engagement needle portion 210 and an output needle portion 212. The engagement needle portion serves to communicate with a drug cartridge of a drug delivery device when the medicated module is attached to the drug delivery device. Specifically, the output needle portion serves as the output needle that a user can use to subcutaneously inject medicament. The output needle serves as an output for a dose of medicament 208 as well as primary medicament from a drug delivery device. It should be noted that although needle 202 is depicted as a double-ended single needle having an engagement portion and an output portion, the engagement needle portion and output needle portion may actually both be separate needles in other embodiments.

The medicated module 200 also includes an attachment means 214 and a needle cover 216. Attachment means 214 is configured to attachment to a corresponding attachment means of a drug delivery device, such as the attachment means 109 at the distal end 132 of device 100. This needle cover 216 may have a connection feature (e.g., a snapfit feature) that allows the cover to be removably attached to the body of the medicated module. Further, the seal 206 is preferably located in the needle cover. In a preferred embodiment, the seal 206 is located at a bottom base at the distal end 224 of the needle cover.

Figure 2 depicts the medicated module 200 prior to the module being attached to a drug delivery device. Prior to attachment, needle 202 partially pierces top seal 204 and bottom seal 206. Specifically, a piercing tip 218 of the engagement needle portion partially pierces the top seal 204, and a piercing tip 220 of the output needle portion 212 partially pierces the bottom seal 206. Because the ends of needle 202 only partially pierce the seals 204, 206, the seals provide a fluid tight seal that prevent leakage of the medicament 208 from the ends of the needle 202. Example materials that the seals could be manufactured from include, but are not limited to, polyvinylidene chloride (PVdCl), LDPE (low density polyethylene), engineering polymers (e.g. Polypropylene - PP, Acrylonitrile Butadiene Styrene - ABS, Cyclo Olefin Polymer - COP, Polyethylene terephthalate - PET, Polycarbonate - PC, Polyoxymethylene - POM, and other like materials), and LLDPE (linear low density polyethylene). Alternatively the membrane may be constructed from a multi-layer foil, such as, but not limited to, PE (Polyethylene) with a PET (Polyethylene terephthalate) coating for example. TPE (Thermoplastic Elastomers), Liquid Silicone Rubber (LSR) and natural rubbers. Where improved barrier properties are desirable, laminate materials may be used e.g. multilayer materials consisting of the primary membrane material (potentially as above) plus additional thin layers of materials like PVC (Polyvinyl chloride) PCTFE (Polychlorotrifluoro ethylene) or Aluminuim.

Further, the size (e.g., width and length) and shape of the seals may vary. The size of a seal is preferably large enough so as to provide an adequate liquid-tight seal when a needle end partially pierces the seal. As an example, a seal may be 5mmx5mm square. Larger seals are possible, such as 1cmx5mm or 1cmx1cm. Other sizes and shapes are possible as well.

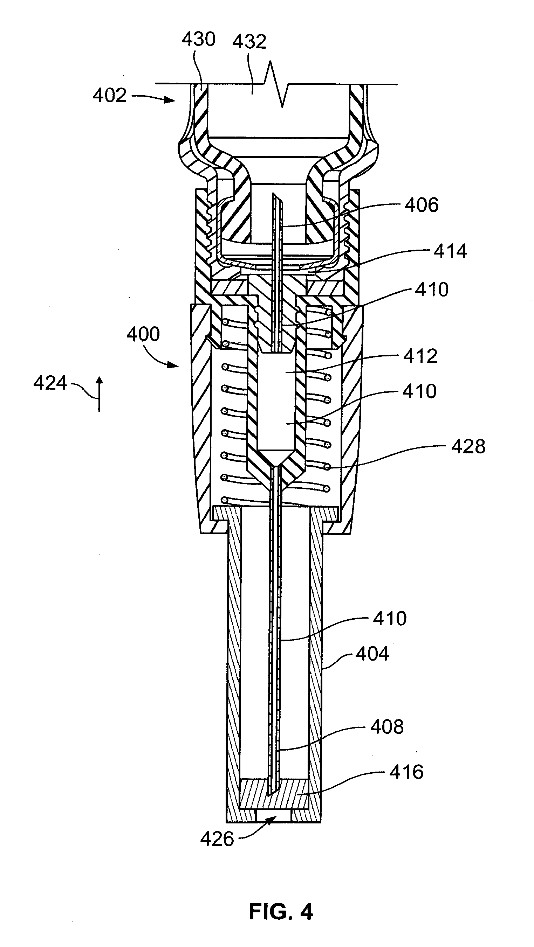

The seals 204, 206 provide a fluid tight engagement for needle 202 until the seals are moved such that the needle no longer partially pierces the seals. The seals may move during the attachment process or prior to the dose delivery (i.e., injection) process. Specifically, the top seal may be moved and fully pierced during attachment of the medicated module to the drug delivery device. Further, the bottom seal may be moved at some point in time prior to injection. In the example of Figures 2-3, the bottom seal is moved distally by the user when the user removes the needle cover. This action withdraws the bottom seal from the needle and exposes the open end of the output needle. However, it should be understood that the bottom seal may be removed from the distal end of the needle in a different way. For example, in the example of Figure 4, the bottom seal is moved in a proximal direction by the force of the retracting needle guard. The output needle then fully pierces the bottom seal, thus exposing the open end of the output needle.

Returning to the example of Figure 2, the top seal 204 could be attached to the tip of engagement needle portion 210 in a variety of ways. For instance, the seal 204 may be a stand-alone seal that may be pushed up or down the needle. However, for added stability, which may be useful during the shipping process and storage, the top seal may be removably secured to an inner wall 226 of the medicated module. This may be accomplished with a material that can stretch and/or break. Preferably, the material may easily stretch and/or break under the force caused by a drug delivery device when it is attached to the medicated module 200.

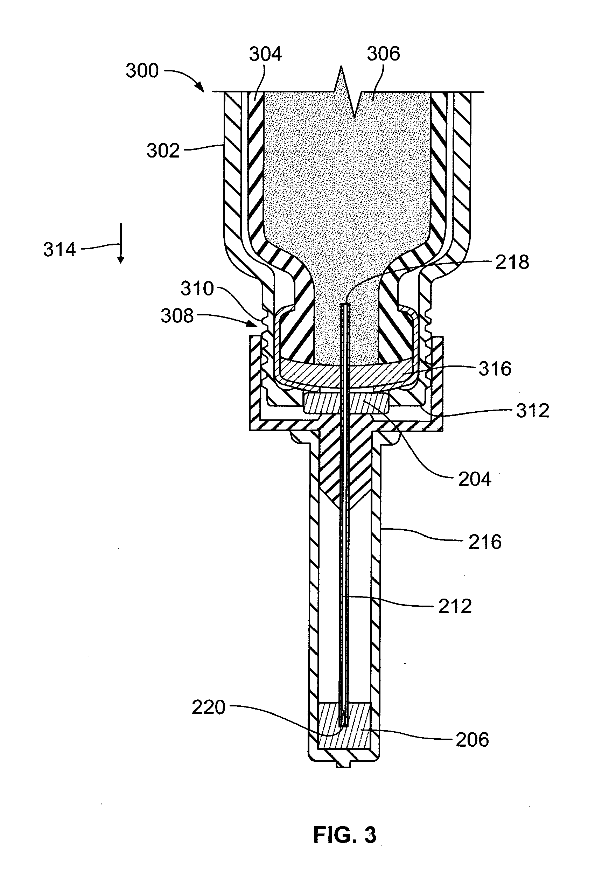

Figure 3 depicts medicated module 200 attached to drug delivery device 300. The drug delivery device includes a cartridge holder 302 and drug cartridge 304, which holds primary medicament 306. The attachment means 308 include threads 310 that engage with the attachment means 214 of the medicated module.

During attachment, the distal end 312 of the cartridge holder contacts the top surface of seal 204 and forces the top seal 204 axially downward in direction 314. Forcing the top seal in direction 314 causes the piercing tip 218 of the engagement needle 210 to fully pierce the top seal. Subsequent to piercing the top seal 204, the piercing tip 218 of the engagement needle 210 pierces the septum 316 of the drug cartridge 304. This action creates fluid communication between the medicament 208 of the medicated module and the primary medicament 306 of the primary device.

Beneficially, piercing the top seal in this manner helps minimize the risk of leakage. Further, piercing the top seal in this manner helps reduce the risk that air is introduced to the system during needle attachment.

As mentioned above, lower seal 206 is preferably contained in the needle cover 216. The needle cover 216 may be removed by the user prior to use (i.e., injection). After removal of the needle cover 216 by the user, the open end 220 of the output needle portion 212 is exposed. The primary medicament 306 and medicament 208 may then be subcutaneously injected. Preferably, the top seal is secured in the bottom of the base of the needle cover, so the bottom seal stays in the needle cover when the cover is removed. The seal may be secured in the needle cover using, for example, adhesive and/or glue.

An alternative embodiment in accordance with Applicants' proposed concept is shown in Figure 4. Figure 4 depicts medicated module 400 attached to primary drug delivery device 402. This embodiment is similar in many respects to medicated module 200. Therefore, many of the options discussed with regard to Figures 2-3 are possible in the option of Figure 4, and vice versa. However, rather than having a needle cover such as needle cover 216, medicated module 400 includes a needle guard 404. The lower seal for sealing the distal end of the output needle is located in the needle guard 404.

Medicated module 400 includes a first needle 406 (i.e., the engagement needle) and a second needle 408 (i.e., the output needle). The engagement needle 406 and the output needle 408 are in fluid communication with one another and hold a medicament 410. In this example, a reservoir 412 is located between and in fluid communication with the engagement needle 406 and the output needle 408. Reservoir 412 may beneficially allow for storage of a large volume of the secondary medicament 410. Similar to the embodiment of Figures 2-3, medicated module 400 includes a first seal 414 (i.e., top seal) and a second seal 416 (i.e., bottom seal). Prior to attachment, the engagement needle 406 partially pierces top seal 414. Therefore, the fluid tight seal prevents leakage of medicament 410 through this proximal end of the engagement needle. Further, output needle 408 partially pierces bottom seal 416. Therefore, the lower seal 316 prevents leakage of medicament 410 through this distal end of the output needle.

As depicted in Figure 4, after medicated module 400 is attached to drug delivery device 402, the top seal 414 is fully pierced by the engagement needle, and this piercing opens up fluid communication between the engagement needle and the drug cartridge 430, which holds primary medicament 432. Thus, when a dose is injected, the primary medicament 432 and secondary medicament 410 from the medicated module may both be injected.

After attaching the medicated module 400 to drug delivery device 402, a user may inject a dose. During insertion of the output needle 408 into the user, the act of insertion forces needle guard 404 axially in direction 424. This relative motion causes the output needle 408 to fully pierce the lower seal 416 immediately prior to piercing the skin of the user. After fully piercing lower seal 416, the output needle travels through opening 426 in order to pierce a user's skin.

This needle-guard arrangement of Figure 4 may be preferred to the needle-cover arrangement of Figures 2-3, as breaking the fluid tight seal provided by lower seal 416 only immediately prior to piercing skin may beneficially further reduce the risk that air is introduced to the medicated needle.

The medicated module may also include a biasing member 428. This biasing member 428 is operably connected to the needle guard 404. The biasing member may serve to keep the needle guard around the needle when a user is not applying an axially directed force on the needle guard. Further, after injection, the biasing member acts to return the needle guard to its pre-injection position surrounding the needle.

Should a user attempt to reuse a previously used a medicated module having the proposed sealing means may include a feature or features that prevent re-attachment to a drug delivery device.

It should be understood that Applicants' proposed sealing features may be used in an medicated module where the drug is essentially contained within a cavity formed between a tip of an engagement needle (i.e., the needle that pierces the septum of a drug cartridge when the medicated module is attached to a drug delivery device) and a tip of an outlet needle (i.e., the needle that is inserted into the patient). Figures 2-4 show possible examples of an arrangement of such a medicated module. However, variations are possible, and it should be understood that the proposed sealing means may be used for other medicated module designs.

Applicants' proposed concept beneficially provides a sealing means to seal medicament in a medicated module. The sealing means beneficially prevents leakage and reduces or minimizes contamination. Further, the sealing means requires few components and provides an effective and inexpensive means to secure medicament in a medicated module.

In the above described embodiments, the medicament in the medicated module may be either in a powdered solid state, any fluid state contained within the needle and/or reservoir, or coated to the inside surface of the needle. The greater concentration of the solid form of the medicament has the benefit of occupying a smaller volume than the liquid having lower concentration. This in turn reduces the ullage of the medicated module. The device would be used in the same manner as the preferred embodiment with the medicament in the medicated module being dissolved by the primary medicament during dispense.

The connection or attachment between the medicated module of the above descried embodiments may contain additional features (not shown), such as connectors, stops, splines, ribs, grooves, and the like design features, that ensure that specific medicated module are attachable only to matching drug delivery devices. Such additional features would prevent the insertion of a non-appropriate medicated module to a non-matching injection device.

The shape of the medicated module may be a cylindrical body or any other geometric shape suitable for defining a fluid reservoir or for containing discrete self-contained reservoir of the medicament in the medicated module and for attaching one or more needle cannula. The medicated module can be manufactured from glass or other drug contact suitable material. The integrated output needle can be any needle cannula suitable for subcutaneous or intramuscular injection. Preferably the medicated module is provided by a drug manufacturer as a stand-alone and separate device that is sealed to preserve sterility. The sterile seal of the module is preferably designed to be opened automatically, e.g. by cutting, tearing or peeling, when the medicated module is advanced or attached to the drug delivery device by the user.

The medicated module of Applicants' concept could be designed to operate in conjunction with a multiple use injection device, preferably a pen-type multi-dose injection device, similar to what is illustrated in Fig. 1. The injection device could be a reusable or disposable device. By disposable device it is meant an injection device that is obtained from the manufacturer preloaded with medicament and cannot be reloaded with new medicament after the initial medicament is exhausted. The device may be a fixed dose or a settable dose and preferably a multi-dose device, however, in some cases it may be beneficial to use a single dose, disposable device.

A typical injection device contains a cartridge or other reservoir of medication. This cartridge is typically cylindrical in shape and is usually manufactured in glass. The cartridge is sealed at one end with a rubber bung and at the other end by a rubber septum. The injection pen is designed to deliver multiple injections. The delivery mechanism is typically powered by a manual action of the user, however, the injection mechanism may also be powered by other means such as a spring, compressed gas or electrical energy.

Exemplary embodiments of the present invention have been described. Those skilled in the art will understand, however, that changes and modifications may be made to these embodiments without departing from the true scope of the present invention, which is defined by the claims.

## Claims

1. A medicated module (200, 400) attachable to a drug delivery device, the medicated module (200, 400) comprising:
a needle (202), wherein the needle (202) holds a medicament (208, 410) and includes an output needle portion (212)
**characterized in that** the medicated module further comprises:
a first seal (204, 414); and
a second seal (206, 416),
wherein the needle (202) includes an engagement needle portion (210), which is in fluid communication with the output needle portion (212), wherein a proximal end of the engagement needle portion (210) partially pierces the first seal (204, 414), and wherein
a distal end of the output needle portion (212) partially pierces the second seal (206, 416).

2. The medicated module (200, 400) of claim 1, wherein the first seal (204, 414) and the second seal (206, 416) each provide a fluid-tight seal that prevents leakage of the medicament (208, 410).

3. The medicated module (200, 400) of claim 1 or 2, further comprising a needle cover (216), wherein the needle cover (216) comprises the second seal (206, 416).

4. The medicated module (200, 400) of claim 3, wherein the second seal (206, 416) is positioned at a base of the needle cover (216).

5. The medicated module (200, 400) of any of the preceding claims, wherein during attachment of the medicated module (200, 400) to the drug delivery device, a distal end of the drug delivery device forces the first seal (204, 414) in a distal direction, wherein the engagement needle portion (210) fully pierces the first seal (204, 414).

6. The medicated module (200, 400) of claim 3, wherein the needle cover (216) may be removed by a user, and wherein, after the needle cover (216) is removed, the second seal (206, 416) is removed from the distal end of the output needle portion (212).

7. The medicated module (200, 400) of any of the preceding claims, further comprising a needle guard (404), wherein the needle guard (404) comprises the second seal (206, 416).

8. The medicated module (200, 400) of claim 7, wherein during injection, the needle guard (404) is forced in the proximal direction, and wherein the output needle portion (212) fully pierces the second seal (206, 416).

9. A medicated module (200, 400) attachable to a drug delivery device, the medicated module (200, 400) comprising:
an output needle (408) holding a medicament (208, 410);
**characterized in that** the medicated module (200, 400) further comprises an engagement needle (406) holding the medicament (208, 410), wherein the engagement needle (406) and the output needle (408) are in fluid communication,
a first seal (204, 414); and
a second seal (206, 416),
wherein a proximal end of the engagement needle (406) partially pierces the first seal (204, 414), and wherein a distal end of the output needle (408) partially pierces the second seal (206, 416).

10. The medicated module (200, 400) of claim 9, further comprising a needle cover (216), wherein the needle cover (216) comprises the second seal (206, 416).

11. The medicated module (200, 400) of claim 10, wherein the second seal (206, 416) is positioned at a base of the needle cover (216).

12. The medicated module (200, 400) of any of claims 9-11, wherein during attachment of the medicated module (200, 400) to the drug delivery device, a distal end of the drug delivery device forces the first seal (204, 414) in a distal direction, wherein the engagement needle (406) fully pierces the first seal (204, 414).

13. The medicated module (200, 400) of any of claims 9-12, further comprising a needle guard (404), wherein the needle guard (404) comprises the second seal (206, 416).

14. The medicated module (200, 400) of any of claims 9-13, further comprising a reservoir (412) located between and in fluid communication with the engagement needle (406) and the output needle (408).

## Patentansprüche

1. Arzneimittelhaltiges Modul (200, 400), das an einer Medikamenten-Verabreichungsvorrichtung befestigbar ist, wobei das arzneimittelhaltige Modul (200, 400) umfasst:
eine Nadel (202), wobei die Nadel (202) ein Arzneimittel (208, 410) enthält und einen Ausgabenadelabschnitt (212) aufweist,
**dadurch gekennzeichnet, dass** das arzneimittelhaltige Modul ferner umfasst:
eine erste Dichtung (204, 414); und
eine zweite Dichtung (206, 416),
wobei die Nadel (202) einen Eingriffsnadelabschnitt (210) aufweist, der in Fluidverbindung mit dem Ausgabenadelabschnitt (212) ist, wobei ein proximales Ende des Eingriffsnadelabschnitts (210) teilweise die erste Dichtung (204, 414) durchsticht und wobei ein distales Ende des Ausgabenadelabschnitts (212) teilweise die zweite Dichtung (206, 416) durchsticht.

2. Arzneimittelhaltiges Modul (200, 400) nach Anspruch 1, wobei die erste Dichtung (204, 414) und die zweite Dichtung (206, 416) jeweils eine fluiddichte Abdichtung vorsehen, die ein Austreten des Arzneimittels (208, 410) verhindert.

3. Arzneimittelhaltiges Modul (200, 400) nach Anspruch 1 oder 2, ferner eine Nadelabdeckung (216) umfassend, wobei die Nadelabdeckung (216) die zweite Dichtung (206, 416) umfasst.

4. Arzneimittelhaltiges Modul (200, 400) nach Anspruch 3, wobei die zweite Dichtung (206, 416) an einem Boden der Nadelabdeckung (216) positioniert ist.

5. Arzneimittelhaltiges Modul (200, 400) nach einem der vorhergehenden Ansprüche, wobei während der Befestigung des arzneimittelhaltigen Moduls (200, 400) an der Medikamenten-Verabreichungsvorrichtung ein distales Ende der Medikamenten-Verabreichungsvorrichtung die erste Dichtung (204, 414) in eine distale Richtung zwingt, wobei der Eingriffsnadelabschnitt (210) vollständig die erste Dichtung (204,414) durchsticht.

6. Arzneimittelhaltiges Modul (200, 400) nach Anspruch 3, wobei die Nadelabdeckung (216) von einem Benutzer entfernt werden kann und wobei, nachdem die Nadelabdeckung (216) entfernt worden ist, die zweite Dichtung (206, 416) von dem distalen Ende des Ausgabenadelabschnitts (212) entfernt wird.

7. Arzneimittelhaltiges Modul (200, 400) nach einem der vorhergehenden Ansprüche, ferner einen Nadelschutz (404) umfassend, wobei der Nadelschutz (404) die zweite Dichtung (206, 416) umfasst.

8. Arzneimittelhaltiges Modul (200, 400) nach Anspruch 7, wobei, während der Injektion, der Nadelschutz (404) in die proximale Richtung gezwungen wird und wobei der Ausgabenadelabschnitt (212) vollständig die zweite Dichtung (206, 416) durchsticht.

9. Arzneimittelhaltiges Modul (200, 400), das an einer Medikamenten-Verabreichungsvorrichtung befestigbar ist, wobei das arzneimittelhaltige Modul (200, 400) umfasst:
eine Ausgabenadel (408), die ein Arzneimittel (208, 410) enthält;
**dadurch gekennzeichnet, dass** das arzneimittelhaltige Modul (200, 400) ferner eine Eingriffsnadel (406) aufweist, die das Arzneimittel (208, 410) enthält, wobei die Eingriffsnadel (406) und die Ausgabenadel (408) in Fluidverbindung stehen,
eine erste Dichtung (204, 414); und
eine zweite Dichtung (206, 416),
wobei ein proximales Ende der Eingriffsnadel (406) teilweise die erste Dichtung (204, 414) durchsticht und wobei ein distales Ende der Ausgabenadel (408) teilweise die zweite Dichtung (206, 416) durchsticht.

10. Arzneimittelhaltiges Modul (200, 400) nach Anspruch 9, ferner eine Nadelabdeckung (216) umfassend, wobei die Nadelabdeckung (216) die zweite Dichtung (206, 416) umfasst.

11. Arzneimittelhaltiges Modul (200, 400) nach Anspruch 10, wobei die zweite Dichtung (206, 416) an einem Boden der Nadelabdeckung (216) positioniert ist.

12. Arzneimittelhaltiges Modul (200, 400) nach einem der Ansprüche 9 - 11, wobei, während der Befestigung des arzneimittelhaltigen Moduls (200, 400) an der Medikamenten-Verabreichungsvorrichtung, ein distales Ende der Medikamenten-Verabreichungsvorrichtung die erste Dichtung (204, 414) in eine distale Richtung zwingt, wobei die Eingriffsnadel (406) vollständig die erste Dichtung (204, 414) durchsticht.

13. Arzneimittelhaltiges Modul (200, 400) nach einem der Ansprüche 9 - 12, ferner einen Nadelschutz (404) umfassend, wobei der Nadelschutz (404) die zweite Dichtung (206, 416) umfasst.

14. Arzneimittelhaltiges Modul (200, 400) nach einem der Ansprüche 9-13, ferner einen Behälter (412) umfassend, der sich zwischen und in Fluidverbindung mit der Eingriffsnadel (406) und der Ausgabenadel (408) befindet.

## Revendications

1. Module médicamenteux (200, 400) pouvant être attaché à un dispositif de distribution de médicament, le module médicamenteux (200, 400) comprenant :
une aiguille (202), dans lequel l'aiguille (202) contient un médicament (208, 410) et comprend une partie d'aiguille d'administration (212),
**caractérisé en ce que** le module médicamenteux comprend en outre :
un premier joint (204, 414) ; et
un deuxième joint (206, 416),
dans lequel l'aiguille (202) comprend une partie d'aiguille d'engagement (210), qui est en communication fluidique avec la partie d'aiguille d'administration (212), dans lequel une extrémité proximale de la partie d'aiguille d'engagement (210) perce partiellement le premier joint (204, 414), et dans lequel une extrémité distale de la partie d'aiguille d'administration (212) perce partiellement le deuxième joint (206, 416).

2. Module médicamenteux (200, 400) selon la revendication 1, dans lequel le premier joint (204, 414) et le deuxième joint (206, 416) fournissent chacun un joint étanche au fluide qui empêche toute fuite du médicament (208, 410).

3. Module médicamenteux (200, 400) selon la revendication 1 ou 2, comprenant en outre un capuchon d'aiguille (216), dans lequel le capuchon d'aiguille (216) comprend le deuxième joint (206, 416).

4. Module médicamenteux (200, 400) selon la revendication 3, dans lequel le deuxième joint (206, 416) est positionné à une base du capuchon d'aiguille (216).

5. Module médicamenteux (200, 400) selon l'une quelconque des revendications précédentes, dans lequel, pendant la fixation du module médicamenteux (200, 400) au dispositif de distribution de médicament, une extrémité distale du dispositif de distribution de médicament force le premier joint (204, 414) dans une direction distale, dans lequel la partie d'aiguille d'engagement (210) perce complètement le premier joint (204, 414).

6. Module médicamenteux (200, 400) selon la revendication 3, dans lequel le capuchon d'aiguille (216) peut être enlevé par un utilisateur, et dans lequel, une fois que le capuchon d'aiguille (216) a été enlevé, le deuxième joint (206, 416) est enlevé de l'extrémité distale de la partie d'aiguille d'administration (212).

7. Module médicamenteux (200, 400) selon l'une quelconque des revendications précédentes, comprenant en outre une protection d'aiguille (404), dans lequel la protection d'aiguille (404) comprend le deuxième joint (206, 416).

8. Module médicamenteux (200, 400) selon la revendication 7, dans lequel, pendant l'injection, la protection d'aiguille (404) est forcée dans la direction proximale, et dans lequel la partie d'aiguille d'administration (212) perce complètement le deuxième joint (206, 416).

9. Module médicamenteux (200, 400) pouvant être attaché à un dispositif de distribution de médicament, le module médicamenteux (200, 400) comprenant :
une aiguille d'administration (408) contenant un médicament (208, 410) ;
**caractérisé en ce que** le module médicamenteux (200, 400) comprend en outre une aiguille d'engagement (406) contenant le médicament (208, 410), dans lequel l'aiguille d'engagement (406) et l'aiguille d'administration (408) sont en communication fluidique,
un premier joint (204, 414) ; et
un deuxième joint (206, 416),
dans lequel une extrémité proximale de l'aiguille d'engagement (406) perce partiellement le premier joint (204, 414), et dans lequel une extrémité distale de l'aiguille d'administration (408) perce partiellement le deuxième joint (206, 416).

10. Module médicamenteux (200, 400) selon la revendication 9, comprenant en outre un capuchon d'aiguille (216), dans lequel le capuchon d'aiguille (216) comprend le deuxième joint (206, 416).

11. Module médicamenteux (200, 400) selon la revendication 10, dans lequel le deuxième joint (206, 416) est positionné à une base du capuchon d'aiguille (216).

12. Module médicamenteux (200, 400) selon l'une quelconque des revendications 9 à 11, dans lequel, pendant la fixation du module médicamenteux (200, 400) au dispositif de distribution de médicament, une extrémité distale du dispositif de distribution de médicament force le premier joint (204, 414) dans une direction distale, dans lequel l'aiguille d'engagement (406) perce complètement le premier joint (204, 414).

13. Module médicamenteux (200, 400) selon l'une quelconque des revendications 9 à 12, comprenant en outre une protection d'aiguille (404), dans lequel la protection d'aiguille (404) comprend le deuxième joint (206, 416).

14. Module médicamenteux (200, 400) selon l'une quelconque des revendications 9 à 13, comprenant en outre un réservoir (412) qui est situé entre et est en communication fluidique avec l'aiguille d'engagement (406) et l'aiguille d'administration (408).
